# FASCICULE DE BREVET EUROPEEN

(11) **EP 0 753 732 B1**
(45) Date de publication et mention de la délivrance du brevet: **21.05.2003**
(21) Numéro de dépôt: 96401234.8
(22) Date de dépôt: 07.06.1996
(51) Int. Cl.: C12M 1/34, G01N 15/14

(54) **Procédé de numération de particules faiblement luminescentes**
Verfahren zum Abzählen leicht lumineszierender Teilchen
Process of counting weakly luminescent particles

(30) Priorité: 12.06.1995 FR 9506923
(43) Date de publication de la demande: 15.01.1997
(73) Titulaire: Chemometec, 3450 Allerod (DK)
(72) Inventeur: Bisconte de Saint Julien, Jean-Claude, 91640 Briis sous Forges (FR)
(74) Mandataire: Hoeiberg, Susanne

(56) Documents cités:
- EP-A- 0 447 034
- EP-A- 0 529 084
- EP-A- 0 647 858

## Description

La présente invention concerne un procédé de numération de particules faiblement luminescentes, en particulier pour la numération de cellules et de micro-organismes marqués avec un marqueur coloré ou fluorescent marquant sélectivement les micro-organismes et cellules à dénombrer.

On connaît dans l'état de la technique la demande de brevet européen EP 0 647 858 A décrivant un procédé de comptage des pulsations de lumière émises par des particules chemo-luminescentes comprises dans un support plat. Le procédé est basé sur l'utilisation d'un dispositif pour intensification de l'image connecté au détecteur d'images, qui doit être situé très près de support. En plus on connaît la demande de brevet européen EP 0 447 034 A décrivant un procédé pour déterminer la résistance de cellules carcinogènes à une drogue, qui consiste à mesurer la croissance d'une culture desdites cellules au moyen de l'acquisation d'images avec un processeur d'images qui est connecté à un instrument optique tel qu'un microscope. On connaît aussi dans l'état de la technique la demande de brevet européen 0 529 858 A décrivant un procédé pour dénombrer les microorganismes présents dans une solution dans lequel les microorganismes sont enlevés sur une membrane de filtration, impregnés avec une substance qui induit la production de luminescence, et dénombrés à l'aide d'un systeme pour analyser des images luminsecentes.

Les procédés selon l'état de la technique consistent à acquérir une image à un fort grossissement et à procéder à une analyse de cette image après des traitements informatiques de reconnaissance de formes, par exemple de reconnaissance de contours. En raison du fort grossissement nécessaire pour obtenir des images détaillées des objets à dénombrer et de la très faible densité de ces objets, il est nécessaire d'explorer un grand nombre de champs sur un échantillon, et de procéder ensuite à une analyse statistique pour déduire la densité d'objets dans l'échantillon.

L'évolution des appareillages automatiques montre que la tendance naturelle de l'homme de métier est de perfectionner continuellement le comptage individuel des événements, en passant du comptage visuel de colonies formées par une culture sur un milieu nutritif dans une boîte de PETRI, à l'association de la filtration sur membrane et du comptage automatique par traitement d'image, puis à l'automatisation du déplacement du microscope en position X-Y et en profondeur de champ pour multiplier le nombre de champs analysés. Une autre approche, également sur un principe de comptage individuel d'événements et connue sous le nom de cytométrie de flux, consiste à compter individuellement le passage d'un événement dans un flux porteur.

Le comptage unitaire des événements n'est possible qu'avec l'utilisation de moyens optiques perfectionnés garantissant une discrimination élevée des objets. On utilise habituellement une caméra vidéo associée à un microscope à fort grossissement permettant d'obtenir une image très fine, couvrant donc une partie infime de l'échantillon. Ces moyens optiques performants - et donc fragiles - sont associés à des moyens mécaniques performants permettant d'assurer des micro-déplacements dans les trois dimensions de la platine porte-échantillon par rapport à l'objectif de manière à permettre le balayage d'un nombre de champs statistiquement significatif. Il est donc incontestable que l'amélioration de la fiabilité se traduit par une sophistication des performances optiques et mécaniques et se traduit par un coût élevé et une utilisation délicate en dehors du laboratoire.

La présente invention va radicalement à l'encontre de cette démarche qui consiste à améliorer les techniques de comptage unitaire des événements, en proposant une acquisition d'une image des micro-organismes et cellules marqués fixés sur un support sous faible grossissement par une caméra à accumulation de photons. Par faible grossissement, on entendra un grossissement sensiblement inférieur à celui d'un microscope habituellement mis en oeuvre pour des dispositifs de comptage unitaire de l'état de la technique, par exemple un grossissement inférieur à 100.

Selon l'invention, on procède à l'aide d'une caméra à accumulation de photons à l'acquisition d'au moins une image I de faible résolution sous un grossissement inférieur à 40, à procéder à un seuillage de ladite image de faible résolution de façon à obtenir au moins une image binaire Ib, à procéder à au moins un filtrage dimensionnel et à décompter le nomber N d'objects appartenant à une classe de dimensions donné dans l'image binaire Ib.

Selon l'invention, on procède à l'aide d'une caméra à accumulation de photons, pendant une intervalle de temps court par rapport à la vitesse d'évolution desdites particules luminescentes, à l'acquisition d'une pluralité d'images Iᵢ correspondant à des temps d'acquisition différentes, à procéder pour chacune des images Iᵢ à un seuillage de façon à obtenir des images binaires Ibᵢ, à procéder à au moins un filtrage dimensionnel et à décompter le nombre Nᵢ d'objets appartenant à une classe de dimensions donnée dans chacune des images binaires Ibᵢ, le nombre de particules luminescentes correspondant à la valeur maximale de Nᵢ.

La caméra à accumulation délivre un signal dont la dynamique est très élevée et qui, associé à un traitement numérique, permet d'obtenir un résultat précis nonobstant la faible résolution. En particulier, l'acquisition par une caméra à accumulation de photons permet de supprimer la focalisation, et de travailler de manière simultanée et homogène sur le champ d'observation.

L'accumulation de photons et la mise en oeuvre du procédé selon l'invention permettent d'obtenir des informations exploitables sur des images de résolution très médiocre, et couvrant donc un champ étendu.

Un faible grossissement, par exemple de 4, permet de compter les photons, mais en aucun cas de reconnaître les bactéries. Le recours à des moyens d'acquisition d'images de faible résolution, sous faible grossissement, qui va à l'encontre de l'enseignement des procédés et installations selon l'état de la technique, présente des avantages essentiels.

En premier lieu, les problèmes de focalisation sont considérablement simplifiés, voire supprimés. Les procédés selon l'état de la technique mettaient en oeuvre des moyens très complexes pour garantir une parfaite focalisation sur le plan passant par les objets à dénombrer, et souvent nécessitaient le balayage de plusieurs plans de focalisation successifs pour un champ d'observation donné. Le temps de comptage s'en trouvait donc considérablement augmenté.

En second lieu, l'acquisition d'images de faible résolution permet d'explorer un champ étendu de l'échantillon, voire la totalité d'un échantillon. Les objets à dénombrer étant généralement de très faible occurrence, cet avantage est déterminant. En effet, les procédés selon l'état de la technique se contentent généralement d'explorer un nombre de champs statistiquement représentatifs, et non pas la totalité de l'échantillon. Pour des phénomènes présentant un écart-type important dû à des artefacts (agglomération de particules, répartition non homogène des objets dans l'échantillon), cette manière de procéder est incontestablement moins satisfaisante que le procédé selon l'invention permettant une acquisition globale sur l'ensemble de l'échantillon.

En troisième lieu, le traitement numérique porte essentiellement sur des images binaires, et consiste essentiellement en une classe des images binaires des objets en fonction de leur taille. Il met en oeuvre des algorithmes simples et ne nécessitant pas de puissance ou de temps de calcul important, contrairement aux procédés selon l'état de la technique, basés sur des méthodes lourdes de reconnaissance de forme et de traitements complexes et gourmands en temps de calcul.

En quatrième lieu, il est possible d'utiliser une source d'excitation de la fluorescence de faible puissance, ce qui évite de "brûler" les marqueurs. Cet avantage est important car en évitant la dégradation des marqueurs au cours de l'opération de dénombrement, on peut conserver les échantillons et procéder ultérieurement à des vérifications de la mesure.

Selon une première variante, le filtrage dimensionnel concerne les objets de l'image binaire occupant une surface supérieure à la section nominale apparente des particules à dénombrer.

Selon une deuxième variante, le filtrage dimensionnel concerne les objets de l'image binaire occupant une surface sensiblement égale à la section nominale apparente des particules à dénombrer.

Selon un mode de mise en oeuvre particulier, on détermine plusieurs classes d'objets en vue du dénombrement de particules de types différents.

L'invention concerne également une installation pour la numération de particules faiblement luminescentes, en particulier installation pour la numération de cellules et de micro-organismes marqués avec un marqueur coloré ou fluorescent marquant sélectivement les micro-organismes et cellules à dénombrer, comportant une source d'excitation des particules luminescentes et une caméra à accumulation de photons associée à une optique de faible grossissement propre à former une image de l'échantillon analysé d'une résolution suffisante pour que l'image apparente des particules à dénombrer corresponde à quelques pixels. La caméra à accumulation est commandé par un microcalculateur de manière à réaliser une pluralité d'images Iᵢ correspondant à des temps d'acquisition différents, le signal fourni par ladite caméra étant analysé de manière à permettre le dénombrement des particules d'un ou de plusieurs type données.

De préférence, le signal délivré par la caméra à accumulation et correspondant à chacune des images Iᵢ est traité par un microcalculateur réalisant un seuillage de façon à obtenir des images binaires Ibᵢ, et à au moins un filtrage dimensionnel et à décompter le nombre Nᵢ d'objets appartenant à une classe de dimensions donnée dans chacune des images binaires Ibᵢ, le nombre de particules luminescentes correspondant à la valeur maximale de Nᵢ.

Selon un mode de réalisation préféré, la caméra à accumulation est associée à une optique d'un grossissement inférieur à 40.

Selon un mode de mise en oeuvre particulier, les échantillons sont portés par une bande défilant de manière séquentielle sous l'optique associé à la caméra à accumulation de photons.

Selon une variante particulière de réalisation, l'installation selon l'invention comporte un dispositif de préparation des échantillons formé par une bande défilant de manière séquentielle, ladite bande étant formée par l'association d'une couche de filtrage et d'un film de protection, ladite bande passant successivement sous un moyen propre à déposer un fluide contenant les particules à dénombrer, sous un moyen propre à délivrer une quantité nominal d'un réactif de marquage, sous un moyen d'aspiration, puis sous le moyen de dénombrement.

L'invention sera mieux comprise dans ce qui suit, à la lecture de la description faisant référence aux dessins annexés où :
- la figure 1 représente une vue en coupe d'un premier exemple de réalisation d'une installation selon l'invention ;
- la figure 2 représente la courbe de l'évolution de la taille apparente de l'objet en fonction du temps d'acquisition ;
- la figure 3 représente un schéma de la séquence du procédé de dénombrement ;
- la figure 4 représente la courbe de la fonction "luminosité fluorescence".

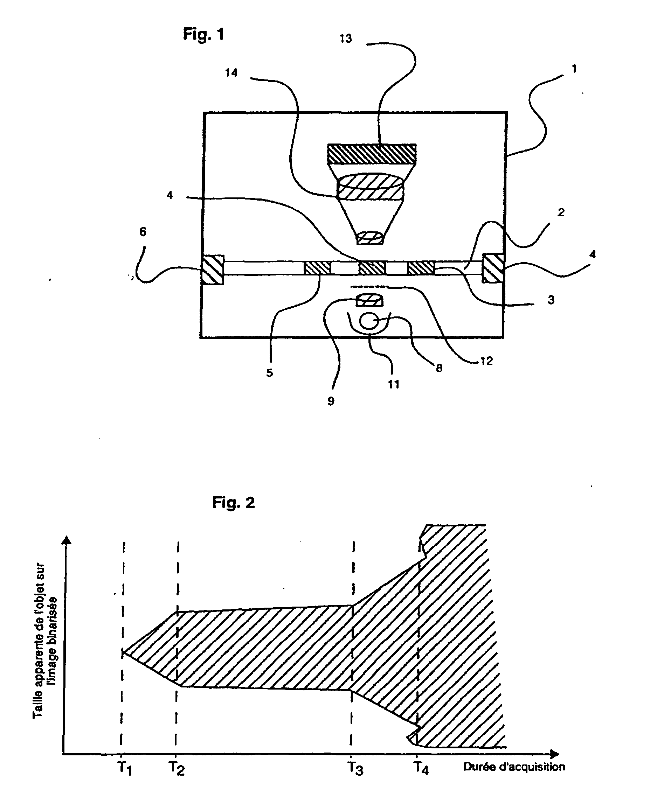

La figure 1 représente une vue schématique, en coupe, d'un exemple de réalisation d'une installation de numération de cellules selon l'invention. La numération s'effectue à partir d'une membrane micro-poreuse présentant une matrice de zones de filtration dont la configuration est déterminée par l'appareil de filtration employé, par exemple un module de filtration commercialisé par la société BIOMERIEUX sous le nom de COBRA (marque déposée).

La préparation de l'échantillon s'effectue de manière connue par filtration et coloration.

L'installation comporte un boîtier (1) rigide et opaque s'ouvrant par une trappe permettant l'introduction d'une membrane micro-poreuse (2) dans un support souple indexé portant des filtrats (3 à 5). Cette membrane (2) est placée sur un support métallique formant un tiroir coulissant entre deux rails de guidages (6, 7).

Une source de rayonnements ultraviolets (8) associée à un condenseur (9) est disposée en dessous du tiroir portant la membrane (2). Un filtre passe-bande (12) laissant passer les rayonnements U.V. dans la longueur d'onde d'excitation des marqueurs est intercalé entre la rampe de sources U.V. (8) et la membrane (2). Un réflecteur métallisé (11) renvoie une partie du rayonnement vers le filtre (12).

Du coté opposé à la rampe U.V., l'installation comporte un capteur à accumulation de photons (13) associé à un bloc optique (14) présentant un grossissement de l'ordre de 10, de préférence compris entre 4 et 40.

Le capteur à accumulation de photons comporte à titre d'exemple 768*562 pixels de 256 bits.

La figure 2 représente la courbe d'évolution de la taille apparente de l'objet sur l'image binarisée, en fonction de la durée d'acquisition.

Pour une durée d'acquisition inférieure à un temps T₁, la caméra à acquisition de photons ne détecte aucun photon, ou un nombre de photons inférieur à la valeur-seuil déterminée pour binariser l'image.

Pour un temps d'acquisition compris entre T₁ et T₂, la dimension de l'objet croit sensiblement proportionnellement avec la durée d'acquisition T. Dans cette plage de temps on trouve un cône d'apparition de l'objet dont la position sur l'image binarisée de la pointe correspond sensiblement au centre de gravité de l'objet luminescent.

Pour un temps d'acquisition compris entre T₂ et T₃, la dimension de l'objet reste sensiblement constante ou faiblement croisante. La taille apparente de la "tache" de lumière correspond sensiblement à la taille apparente de l'objet luminescent. On désignera par la suite cette zone par "cône nominal".

Cette plage de temps d'acquisition est relativement étendue, et correspond à la plage de temps pour laquelle le dénombrement des objets binarisés entrant dans une classe de dimension pré définie sera représentatif du nombre d'objets luminescents dans l'échantillon.

Pour un intervalle de temps d'acquisition compris entre T₃ et T₄, la dimension de l'objet apparent croit rapidement, du fait de phénomènes de diffusion de la lumière émis par l'objet de l'échantillon. On trouve dans cette plage un cône de diffusion à pente forte.

Au-delà d'un temps d'acquisition T₄, la dimension de la tache croit très rapidement jusqu'à atteindre un seuil de saturation du capteur. Cette zone correspond à la luminosité du fond, qui est détectable pour de très grandes durées d'acquisition, et aux phénomènes de bruits intrinsèques à la caméra d'acquisition.

La durée optimale d'acquisition variera d'un objet à l'autre en fonction de l'intensité de la luminosité. Toutefois, la fourchette de temps d'acquisition comprise entre T₂ et T₃ est suffisamment importante pour contenir, sur une au moins des images Iᵢ aussi bien le début du cône nominal des objets de très faible luminosité que la fin du cône nominal des objets très luminescents.

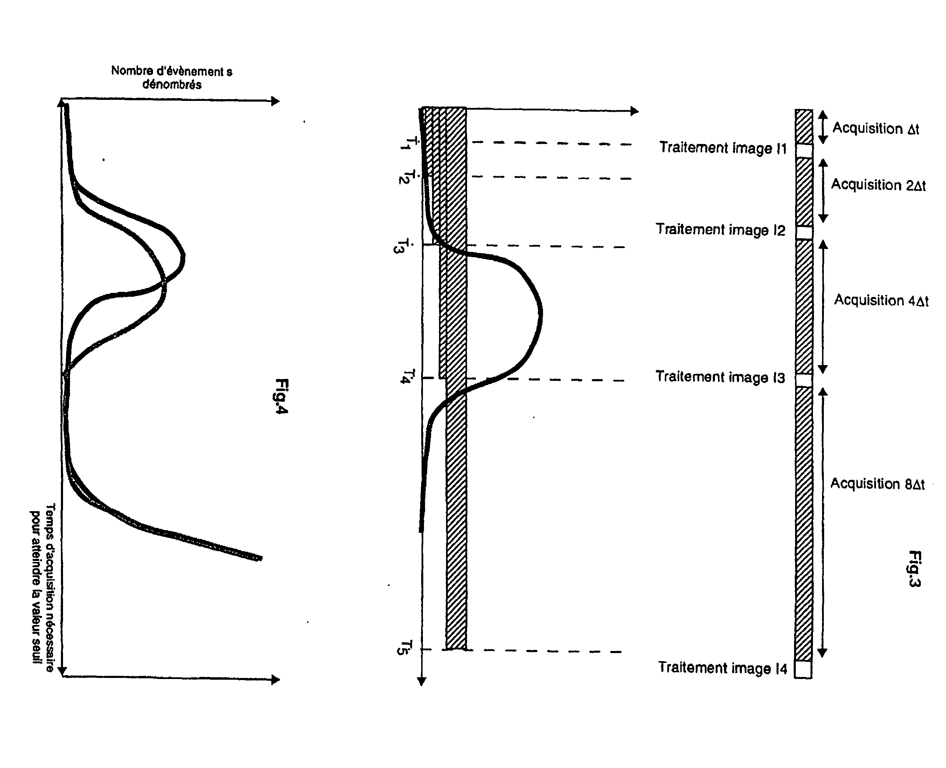

A cet effet, on procède comme schématisé en figure 3 à une "salve" d'acquisition pendant des durées de temps différentes, par exemple croisantes. La progression des durées d'acquisition n'est pas critique. Elle pourra être une progression géométrique de façon à optimiser le temps nécessaire à l'obtention d'une image satisfaisante. La durée totale de la salve d'acquisitions sera brève au regard de l'évolution des phénomènes observés, notamment de l'affaiblissement de la luminescence, ou de la variation de l'éclairage d'excitation.

Le filtrage consiste en un traitement numérique de l'image binarisée. On décompte tout les objets de l'image binarisée dont la dimension, c'est-à-dire le nombre de pixels adjacents, vérifie une fonction déterminée.

Cette fonction est par exemple la fonction "supérieure à N pixels" où N pixels correspond à la valeur moyenne de la section apparente d'un objet à dénombrer, ou encore "compris entre N-ε et N+ε pixels".

Le résultat se traduit par une fonction représentée de manière schématique sur la figure 3, correspondant à une gaussienne.

Le traitement de l'image délivrée après chaque acquisition peut être réalisé en temps réel, ou en différé, chaque image Ii étant alors stockée en mémoire en vue d'un traitement de chacune des images mémorisées à la fin de la salve d'acquisition.

La figure 4 représente la courbe représentative de la durée d'acquisition nécessaire pour atteindre la valeur-seuil Vs mise en oeuvre pour la binarisation de l'image acquise, en fonction de la luminosité des objets.

On trouve une première zone en forme de cloche et une partie divergente correspondant à la luminosité du fond. La partie en forme de cloche se déplace en direction de la partie divergente lorsque la fluorescence des objets est plus petite (courbe en gris).

L'invention concerne selon une variante particulière une installation de contrôle en continu.

Les échantillons sont déposés sur un film déroulant formé par une couche d'un matériau support, par exemple une matière plastique tel que du polyéthylène, sur laquelle est déposée une seconde couche d'un film formant filtre. Un film de protection supérieur est perforé périodiquement pour présenter des zones de filtrage en forme de disque.

Le film est enroulé sur une bobine. Il est déroulé séquentiellement de manière à ce qu'une zone de filtrage passe successivement sous un moyen de dépose d'un échantillon, sous un ou plusieurs moyens de dépose de réactifs, puis sur une zone d'aspiration propre à assurer la concentration des objets à dénombrer sur le filtre, puis sous une zone d'observation. Le film support est séparé du film de filtration avant le passage dans la zone d'aspiration.

Le film est enroulé sur une bobine. Il est déroulé séquentiellement de manière à ce qu'une zone de filtrage passe successivement sous un moyen de dépose d'un échantillon, sous un ou plusieurs moyens de dépose de réactifs, puis sur une zone d'aspiration propre à assurer la concentration des objets à dénombrer sur le filtre, puis sous une zone d'observation. Le film support est séparé du film de filtration avant le passage dans la zone d'aspiration.

## Revendications

1. Procédé de numération de particules faiblement luminescentes, en particulier procédé de numération de cellules et de micro-organismes marqués avec un marqueur coloré ou fluorescent marquant sélectivement les micro-organismes et cellules à dénombrer, **caractérisé en ce que** l'on procède à l'aide d'une caméra à accumulation de photons à l'acquisition d'au moins une image I de faible résolution sous un grossissement inférieur à 40, à procéder à un seuillage de ladite image de faible résolution de façon à obtenir au moins une image binaire Ib, à procéder à au moins un filtrage dimensionnel et à décompter le nombre N d'objects appartenant à une classe de dimensions donné dans l'image binaire Ib.

2. Procédé selon la revendication 1, **caractérisé en ce que** l'on procède à l'aide d'une caméra à accumulation de photons, pendant une intervalle de temps court par rapport au temps d'évolution de la taille apparente de l'object sur l'image binarisée desdites particules luminescentes, à l'acquisition d'une pluralité d'images Iᵢ correspondant à des temps d'aquisisation différentes, à procéder pour chacune des images Iᵢ à un seuillage de façon à obtenir des images binaires Ibᵢ, à procéder à au moins un filtrage dimensionnel et à décompter le nombre Nᵢ d'objects appartenant à une classe de dimensions donnée dans chacune des images binaires Ibᵢ, le nombre de particules luminescentes correspondant à la valeur maximale de Nᵢ.

3. Procédé selon la revendication 2, **caractérisé en ce que** le filtrage dimensionnel concerne les objets de l'image binaire occupant une surface supérieure à la section nominale apparente des particules à dénombrer.

4. Procédé selon la revendication 2, **caractérisé en ce que** le filtrage dimensionnel concerne les objets de l'image binaire occupant une surface correspondant à la section nominale apparente des particules à dénombrer.

5. Procédé selon l'une quelconque des revendications précédentes, **caractérisé en ce que** l'on détermine plusieures classes d'objets en vue du dénombrement de particules de types différents.

6. Procédé selon l'une quelconque des revendications précédentes, **caractérisé en ce que** l'on procède avec un grossissement entre 4 et 40.

7. Procédé selon l'une quelconque des revendications précédentes, **caractérisé en ce que** l'on procede avec un grossissement de l'ordre de 10.

8. Installation pour la numération de particules faiblement luminescentes, en particulier installation pour la numération de cellules et de micro-organismes marqués avec un marqueur coloré ou fluorescent marquant sélectivement les micro-organismes et cellules à dénombrer, **caractérisé en ce qu'**elle comporte une source d'excitation des particules luminescentes et une caméra à accumulation de photons associée à une optique d'un grossissement inferieur à 40 propre à former une image de l'échantillon analysé d'une résolution suffisante pour que l'image apparente des particules à dénombrer corresponde à quelques pixels, ladite caméra à accumulation étant commandé par un microcalculateur de manière à réaliser une pluralité d'images Iᵢ correspondant à des temps d'acquisition différentes, le signal délivré par la caméra à accumulation et correspondant à chacune des images Iᵢ étant traité par un microcalculateur réalisant un seuillage de façon à obtenir des images binaires Ibᵢ, et à au moins un filtrage dimensionnel et à décompter le nombre Nᵢ d'objects appartenant à une classe de dimensions donnée dans chacune des images binaires Ibᵢ le nombre de particules luminescentes correspondant à la valeur maximale de Nᵢ.

9. Installation selon la revendication 8, **caractérisé en ce que** le grossissement est entre 4 et 40.

10. Installation selon la revendication 9, **caractérisé en ce que** le grossissement est de l'ordre de 10.

11. Installation selon l'une quenconques des revendications 8 à 10, **caractérisé en ce que** les échantillons sont portés par une bande défilant de manière séquentielle sous l'optique associé à la caméra à accumulation de photons.

12. Installation selon la revendication 11, **caractérisé en ce qu'**elle comporte un dispositif de préparation des échantillons formé par une bande défilant de manière séquentielle, ladite bande étant formée par l'association d'une couche de filtrage et d'un film de protection, ladite bande passant successivement sous un moyen propre à déposer un fluide contenant les particules à dénombrer, sous un moyen propre à délivrer une quantité nominale d'un réactif de marquage, sous un moyen d'aspiration, puis sous le moyen de dénombrement.

## Patentansprüche

1. Verfahren zum Abzählen leicht lumineszierender Teilchen, insbesondere Verfahren zum Abzählen von Zellen und Mikro-Organismen, die mit einem farbigen oder fluoreszierenden Marker markiert sind, der wahlweise die Mikro-Organismen und Zellen, die abzuzählen sind, markiert, **dadurch gekennzeichnet, dass** man mit Hilfe einer Kamera zum Sammeln von Photonen das Aufbauen wenigstens eines Bildes I von schwacher Auflösung unter einer Vergrößerung von weniger als 40 vornimmet, eine Schwellwertbildung des Bildes mit schwacher Auflösung vornimmt, um wenigstens ein binäres Bild Ib zu erhalten, wenigstens eine Größenfiltrierung und das Abzählen der Anzahl N von Objekten vornimmt, die einer Größenklasse zugehörig ist, die in dem binären Bild Ib gegeben ist.

2. Verfahren nach Anspruch 1, **dadurch gekennzeichnet, dass** ein Schritt des Sammelns von Photonen mit Hilfe einer Kamera durchgeführt wird, während eines kurzen Zeitintervalls bezogen auf die Entwicklungszeit der erscheinenden Größe des Objekts auf dem binärisierten Bild der lumineszierenden Teilchen, eine Mehrzahl von Bildern Iᵢ, die unterschiedlichen Erfassungszeiten entsprechen, erfaßt werden, für jedes dieser Bilder Iᵢ eine Schwellenwertbildung derart durchgeführt wird, dass binäre Bilder Ibᵢ erhalten werden, wenigstens eine Größenfiltrierung durchgeführt wird und die Anzahl Nᵢ von Objekten gezählt wird, die zu einer Größenklasse gehört, die in einem jeden der binären Bilder Ibᵢ gegeben ist, wobei die Anzahl der lumineszierenden Teilchen dem maximalen Wert von Nᵢ entspricht.

3. Verfahren nach Anspruch 2, **dadurch gekennzeichnet, dass** die Größenfiltrierung die binären Bildgegenstände betrifft, die eine Oberfläche besetzen, die größer ist, als der nominale erscheinende Querschnitt der zu zählenden Teilchen.

4. Verfahren nach Anspruch 2, **dadurch gekennzeichnet, dass** die Größenfiltrierung die binären Bildgegenstände betrifft, die eine Oberfläche besetzen, die dem erscheinenden nominalen Querschnitt der zu zählenden Teilchen entspricht.

5. Verfahren nach einem der vorhergehenden Ansprüche, **dadurch gekennzeichnet, dass** mehrere Objektklassen bestimmt werden, im Hinblick auf das Abzählen von Teilchen von unterschiedlichem Typ.

6. Verfahren nach einem der vorhergehenden Ansprüche, **dadurch gekennzeichnet, dass** eine Vergrößerung zwischen 4 und 40 vorgenommen wird.

7. Verfahren nach einem der vorhergehenden Ansprüche, **dadurch gekennzeichnet, dass** eine Vergrößerung in der Größenordnung von 10 vorgenommen wird.

8. Vorrichtung zum Abzählen von leicht lumineszierenden Teilchen, insbesondere Vorrichtung zum Abzählen von Zellen und Mikro-Organismen, die mit einem farbigen oder fluoreszierenden Marker markiert sind, der wahlweise die Mikro-Organismen und die Zellen, die zu zählen sind, markiert, **dadurch gekennzeichnet, dass** sie eine Anregungsquelle der lumineszierenden Teilchen und eine Kamera zum Sammeln von Photonen aufweist, die einer Optik einer Vergrößerung geringer als 40 zugeordnet ist, die in der Lage ist ein Bild der analysierten Probe mit einer ausreichenden Auflösung zu bilden, damit das erscheinende Bild der zu zählenden Teilchen einigen Pixels entspricht, wobei die Sammelkamera durch einen Mikrorechner derart gesteuert wird, dass eine Mehrzahl von Bildern Iᵢ erzeugt wird, die unterschiedlichen Erfassungszeiten entsprechen, wobei das durch die Sammelkamera und einem jeden der Bilder Iᵢ entsprechende Signal von einem Mikrorechner verarbeitet wird, der eine Schwellenwertbildung derart verwirklicht, dass die binären Bilder Ibᵢ erhalten werden, und wenigstens eine Größenfiltrierung und Abzählung der Anzahl von Nᵢ Objekten durchgeführt wird, die zu einer Größenklasse gehören, die in einem jeden der binären Bilder Ibᵢ gegeben sind, wobei die Anzahl der lumineszierenden Teilchen einem maximalen Wert von Nᵢ entspricht.

9. Vorrichtung nach Anspruch 8, **dadurch gekennzeichnet, dass** die Vergrößerung zwischen 4 und 40 beträgt.

10. Vorrichtung nach Anspruch 9, **dadurch gekennzeichnet, dass** die Vergrößerung in der Größenordnung von 10 liegt.

11. Vorrichtung nach einem der Ansprüche 8 bis 10, **dadurch gekennzeichnet, dass** die Proben von einem Band getragen werden, das sich auf sequentielle Weise unter der Optik bewegt, die zur Photonensammelkamera gehört.

12. Vorrichtung nach Anspruch 11, **dadurch gekennzeichnet, dass** sie eine Vorrichtung zum Vorbereiten der Proben aufweist, die von einem Band gebildet wird, das sich auf sequentielle Weise bewegt, wobei das Band durch das Zusammensetzen einer Filtrierschicht und eines Schutzfilmes gebildet wird, wobei das Band nacheinander unter einer Einrichtung, die geeignet ist, ein Fluid abzusetzen, das die zu zählenden Teilchen enthält, unter einer Einrichtung, die in der Lage ist, eine nominale Menge eines Markierungsreagens zu liefern, unter einer Absaugeinrichtung und sodann unter einer Zähleinrichtung bewegt wird.

## Claims

1. Method of counting faintly luminescent particles, in particular a method of counting cells and micro-organisms marked with a coloured or fluorescent marker selectively marking the micro-organisms or cells to be counted, **characterised by** the use of a photon-accumulating camera to obtain at least one image I of low resolution under a magnification of less than 40, thresholding the said low resolution image so as to obtain at least one binary image Ib, providing at least one dimensional filtering and counting the number N of objects belonging to a class of dimensions in the binary image Ib.

2. Method according to claim 1, **characterised by** the use of a photon-accumulating camera for a short interval relative to the development time of the apparent size of the object in the binary image of the said luminescent particles, to obtain a plurality of images I, corresponding to the different data capture times, thresholding each of the images Iᵢ so as to obtain binary images Ibᵢ, providing at least one dimensional filtering and counting the number Nᵢ, of objects belonging to a class of dimensions given in each of the binary images Ibᵢ, with the number of luminescent particles corresponding to a maximum value of Nᵢ.

3. Method according to claim 2, **characterised in that** dimensional filtering concerns the objects of the binary image occupying an area greater than the apparent nominal section of particles to be counted.

4. Method according to claim 2, **characterised in that** dimensional filtering concerns the objects of the binary image occupying an area corresponding to the apparent nominal section of the particles to be counted.

5. Method according to any of the previous claims, **characterised in that** several classes of object are determined with a view to counting different types of particles.

6. Method according to any of the preceding claims, **characterised in that** a magnification of between 4 and 40 is carried out.

7. Method according to any of the preceding claims, **characterised in that** a magnification in the order of 10 is carried out.

8. Apparatus for counting faintly luminescent particles, in particular an apparatus for counting cells and micro-organisms marked with a coloured or fluorescent marker selectively marking the micro-organisms and cells to be counted, **characterised in that** it comprises a source for agitating luminescent particles and a photon-accumulating camera which is connected to a lens with a magnification of less than 40, suitable for forming an image of an analysed sample with sufficient resolution so that the apparent image of the particles to be counted corresponds to several pixels, the said accumulating camera being controlled by a micro-computer in such a way so as to produce a plurality of images I, corresponding to the different data capture times, the signal delivered by the accumulating camera and corresponding to each of the images I being processed by a micro-computer producing thresholding in order to obtain binary images Ibᵢ, at least one dimensional filtering and the counting of the number Nᵢ of objects belonging to a class of dimensions given in each of the binary images Ibᵢ, with the number of luminescent particles corresponding to a maximum value of Nᵢ.

9. Apparatus according to claim 8, **characterised in that** magnification is between 4 and 40.

10. Apparatus according to claim 9, **characterised in that** magnification is in the order of 10.

11. Apparatus according to any of claims 8 to 10, **characterised in that** the samples are carried by a strip passing sequentially under the lens connected to the photon-accumulating camera.

12. Apparatus according to claim 11, **characterised in that** it comprises a device for preparing samples formed by a strip passing sequentially, said strip consisting of a combination of a filtering layer and a protective film, said strip passing successively under a means suitable for depositing a fluid containing particles to be counted, under a means suitable for delivering a nominal quantity of a marking reagent, under a vacuum means, then under the counting means.
